# EUROPÄISCHE PATENTANMELDUNG

(11) **EP 2 090 324 A1**
(43) Veröffentlichungstag der Anmeldung: **19.08.2009**
(21) Anmeldenummer: 08151420.0
(22) Anmeldetag: 14.02.2008
(51) Int. Cl.: A61L 2/26, A61M 5/00, B65B 55/02

(54) **Transfercontainer für pharmazeutische Behältnisse**

(71) Anmelder: Roche Diagnostics GmbH, 68305 Mannheim (DE); F. Hoffmann-La Roche AG, 4070 Basel (CH)
(72) Erfinder: Die Erfindernennung liegt noch nicht vor
(74) Vertreter: Hörschler, Wolfram Johannes

(57) **Zusammenfassung**

Es wird ein wiederverwendbarer Container (110) für den sterilen Transfer pharmazeutischer Behältnisse (112), insbesondere von Spritzenkörpern (114), vorgeschlagen. Der Container (110) umfasst eine Außenhülle (116) und einen sterilisierbaren Innenraum (118, 124), wobei der Innenraum (118, 124) mikrobiell gegen die Umgebung des Containers (110) isoliert ist. Der Innenraum (118, 124) weist mindestens eine Halterung (126) für die Aufnahme mindestens eines Nests (128) zur Lagerung einer Mehrzahl von pharmazeutischen Behältnissen (112) auf. Das Nest (128) ist herausnehmbar in der Halterung (126) aufnehmbar. Der Container (110) weist weiterhin mindestens eine Containertür (170) zum Herausnehmen oder Einführen des Nests (128) in den Innenraum (118, 124) und mindestens eine Schleuse (176) zum Ankoppeln des Containers (110) an einen Sterilraum (180) auf. Die Schleuse (176) ist derart ausgestaltet, dass nach dem Ankoppeln an den Sterilraum ein Öffnen der Containertür (170) ohne wesentliche Unterbrechung der Sterilität ermöglicht wird.

## Beschreibung

### Gebiet der Erfindung

Die Erfindung betrifft einen wiederverwendbaren Container für den Transfer steriler pharmazeutischer Behältnisse. Weiterhin betrifft die Erfindung ein Transfersystem für den sterilen Transfer pharmazeutischer Behältnisse sowie ein Verfahren zum Befüllen pharmazeutischer Behältnisse mit mindestens einem pharmazeutischen Medium. Derartige Container, Transfersysteme und Verfahren lassen sich insbesondere im Bereich der Herstellung und/oder Abfüllung pharmazeutischer Behältnisse, wie beispielsweise Medikamenten, Diagnostika oder Ähnlichem, einsetzen. Die Erfindung ist jedoch grundsätzlich auch in anderen Bereichen einsetzbar, in welchen ein Transfer von keimarmen oder sterilen Behältnissen jedweder Art erforderlich ist, beispielsweise im Bereich der Feinchemikalien oder im Bereich der Lebensmittelindustrie.

### Stand der Technik

In vielen Bereichen der Naturwissenschaft und Technik spielt der Transfer von Behältnissen unter Sterilbedingungen eine Rolle. Als Beispiele sind hier, wie oben bereits aufgeführt, die pharmazeutische Produktion bzw. Abfüllung zu nennen, bei welcher Medizinprodukte (zum Beispiel Diagnostika und/oder therapeutische Medien) in geeignete Behältnisse eingefüllt werden. Ein weiterer möglicher Anwendungsbereich ist die Behandlung bzw. Handhabung biologischer Proben. Weitere Bereiche sind die Nahrungsmittelindustrie, in welchen ebenfalls zumindest eine Keimarmut erforderlich ist. Ohne Beschränkung möglicher weiterer Anwendungen der Erfindung wird im Folgenden die pharmazeutische Produktion beschrieben.

In der pharmazeutischen Industrie wird zur Herstellung insbesondere von befüllten Einmalspritzen, sogenannten Fertigspritzen, unter aseptischen, d. h. zumindest weitgehend keimfreien Bedingungen eine Vielzahl von Abfülltechnologien eingesetzt. Grundsätzlich werden dabei sogenannte Line-Filler-Technologien und sogenannte Tray-Filler-Technologien unterschieden, wobei jedoch auch weitere Abfülltechniken eingesetzt werden können. Die vorliegende Erfindung bezieht sich insbesondere auf Tray-Filler-Technologien, ist jedoch auch für andere Abfülltechnologien einsetzbar, beispielsweise die Line-Filler-Techno lo gie.

Bei der Tray-Filler-Technologie werden unbefüllte, zunächst nicht sterile Spritzenkörper (sogenannte Ready-to-Fill-Spritzenkörper) bei einem Packmittellieferanten in sogenannte Trays, welche üblicherweise aus Kunststoff bestehen, eingebracht. Die Trays sind dabei üblicherweise als nach oben offene Schalen mit hochgezogenem Rand ausgebildet. Diese Trays sind mit innenliegenden Kunststoff-Racks (auch als Nest bezeichnet) bestückt, in die die zuvor gereinigten und an ihrer Spitze verschlossenen Spritzenkörper mit der Spitze nach unten hängend eingesetzt werden. Die Trays mit den Racks und den Spritzenkörpern werden dann in der Regel mit mehreren Lagen von losen bzw. mit dem Tray (einer Art Schale) verschweißten Folien bedeckt bzw. verschlossen und anschließend in der Regel mit Ethylenoxid oder gegebenenfalls anderen Verfahren sterilisiert. Durch die mehrfache Abdeckung bzw. Verschließung wird eine mikrobielle Kontamination nach der Sterilisation unterbunden. Die derart in den Trays verpackten, sterilen Spritzenkörper werden dann beim Arzneimittelhersteller und/oder den Abfüllbetrieben angeliefert. Dort werden die Trays zur Befüllung der Spritzenkörper unter aseptischen Reinraum- und Handhabungs-Bedingungen geöffnet und die Spritzenkörper in der Regel mitsamt Tray und Rack auf eine spezielle Abfüllmaschine (Tray-Filler-Maschinen) verbracht. Die Spritzen werden dort dann befüllt und nach Befüllung - in der Regel noch im Tray - verschlossen. Die gefüllten, verschlossenen Spritzen werden anschließend beim sogenannten "de-nesting" in der Regel maschinell aus den Trays entnommen und weiter prozessiert. Die Trays und Racks verbleiben zurück, ebenso wie die Schutzfolien, welche in der Regel allesamt nicht weiterverwendet werden und einen erheblichen Abfall darstellen.

Ein Beispiel, welches derartige übliche Abfülltechnologien beschreibt, stellt die DE 44 19 475 A1 dar. In dieser Schrift werden Verfahren beschrieben, welche sich insbesondere auf die Tray-Filler-Technologie beziehen, so dass für weitere Details möglicher Abfüllprozesse und der Tray-Filler-Technologie auf diese Druckschrift verwiesen werden kann. Die DE 44 19 475 A1 offenbart eine Einrichtung zum Entfernen der Abdeckung von Magazinkästen, wie sie beispielsweise als Trays in der Tray-Filler-Technologie eingesetzt werden können.

Die DE 44 19 475 A1 macht jedoch bereits teilweise die Nachteile bekannter Technologien sichtbar. So ist die Handhabung der bislang verwendeten, verschweißten und mit mehreren Schutzfolien ausgestatteten Trays im großtechnischen Maßstab äußerst aufwendig. Es müssen spezielle Vorrichtungen, wie beispielsweise die in der DE 44 19 475 A1 beschriebene Vorrichtung, eingesetzt werden, um die Umverpackungen der sterilisierten Spritzenkörper zu öffnen und um die Nester aus den Trays herauszunehmen.

Weiterhin besteht ein hoher Bedarf an Lagerplatz für die mit noch unbefüllten Spritzenkörpern (oder gegebenenfalls anderen Primärpackmitteln) bestückten Trays. Weiterhin sind spezielle Abfüllmaschinen erforderlich (Tray-Filler), welche eigens für die Befüllung der Spritzenkörper, welche in den Trays zur Abfüllstation gelangen, eingerichtet sind.

Ein weiterer Nachteil des oben beschriebenen Verfahrens, welcher zunehmend an Bedeutung gewinnt, ist die hohe Menge an bei diesem Verfahren anfallenden Abfällen. So stellen die Einweg-Umverpackungen, welche nach dem Entnehmen der Spritzenkörper bzw. der Trays entsorgt werden, wertvolle Rohstoffe dar, welche in der Regel ungenutzt vergeudet werden. Dies stellt nicht nur einen erheblichen Kostenfaktor dar, sondern kann bei Abfüllungen im großtechnischen Maßstab auch erhebliche Umweltprobleme bereiten.

### Aufgabe der Erfindung

Aufgabe der vorliegenden Erfindung ist es daher, eine Vorrichtung und ein Verfahren bereitzustellen, welches die Nachteile der aus dem Stand der Technik bekannten Vorrichtungen und Verfahren vermeidet. Insbesondere soll erfindungsgemäß ein steriler Transfer pharmazeutischer Behältnisse von einem ersten Sterilraum in einen zweiten Sterilraum ermöglicht werden, ohne dass die sterile Atmosphäre bei diesem Transfer wesentlich unterbrochen wird, wobei gleichzeitig die bei dem Transfer entstehenden Abfälle zumindest weitgehend reduziert werden sollen.

### Beschreibung der Erfindung

Diese Aufgabe wird durch die Erfindung mit den Merkmalen der unabhängigen Ansprüche gelöst. Vorteilhafte Weiterbildungen der Erfindung sind in den Unteransprüchen aufgeführt. Der Wortlaut sämtlicher Ansprüche wird hiermit durch Bezugnahme zum Inhalt dieser Beschreibung gemacht.

Ein Grundgedanke der vorliegenden Erfindung besteht darin, dass das Dilemma, einerseits bei Abfülllinien auf eine aufwendige Sterilisation der pharmazeutischen Behältnisse verzichten zu wollen und andererseits eine Sterilität am Ort der Abfüllung garantieren zu müssen, dadurch gelöst werden kann, dass ein geeigneter, wieder verwendbarer Container eingesetzt wird. Durch die Wiederverwendbarkeit des Containers, welche beispielsweise durch die Auswahl geeigneter, mehrfach sterilisierbarer und den mechanischen Belastungen beim Transfer gewachsenen Materialien erfolgen kann, wird auch eine Vermeidung von Abfällen durch Einwegverpackungen bewirkt.

Es wird daher ein wieder verwendbarer Container für den sterilen Transfer pharmazeutischer Behältnisse, insbesondere von Spritzenkörpern, vorgeschlagen. Die pharmazeutischen Behältnisse können beispielsweise Spritzenkörper, Ampullen, Phiolen, Flaschen, Gläser oder ähnliche Behältnisse beinhalten, welche für die Aufnahme fester und/oder gasförmiger und/oder flüssiger Inhaltsstoffe geeignet sind. Wie oben dargestellt, umfasst der Begriff "pharmazeutisch" in diesem Zusammenhang sowohl diagnostische Medien als auch Medien mit Wirkstoffen zur Herbeiführung mindestens einer medizinischen Wirkung. Die Erfindung, insbesondere der vorgeschlagene Container, ist jedoch grundsätzlich nicht auf pharmazeutische Behältnisse beschränkt.

Der Container umfasst mindestens eine Außenhülle mit einem sterilisierbaren Innenraum. Unter "sterilisierbar" ist hierbei zu verstehen, dass dieser eine zumindest weitgehende Resistenz gegenüber mindestens einem gängigen Sterilisierverfahren aufweist. Insbesondere kann dies eine Resistenz gegenüber thermischer Sterilisation (beispielsweise bis hinauf zu einer vorgegebenen Mindesttemperatur, beispielsweise einer Temperatur oberhalb von 80 °C) mittels Heißluft und/oder Heißdampf sein. Beispielsweise kann eine typische Heißluftsterilisation eine Beaufschlagung von Temperaturen im Bereich von 180°C bis 340°C umfassen. Alternativ oder zusätzlich kann dies auch eine zumindest weitgehende Resistenz gegen eine Sterilisation durch Strahlenquellen (zum Beispiel elektromagnetische Strahlenquellen, insbesondere UV-Licht, und/oder radioaktiven Strahlenquellen, wie beispielsweise Gamma-Strahlenquellen) oder eine Resistenz gegenüber geeigneten Chemikalien für die Sterilisation, beispielsweise mittels Gasen oder anderen fluiden Medien, zum Beispiel Ethylenoxid, Wasserstoffperoxid, Formaldehyd oder Ähnlichem. Vorzugsweise ist diese zumindest teilweise Resistenz derart ausgestaltet, dass eine Vielzahl von derartigen Sterilisationszyklen möglich ist, ohne dass der Container (d. h. die Außenhülle und/oder im Inneren des Containers aufgenommene Bestandteile, beispielsweise ein Innencontainer) eine Veränderung (beispielsweise Oberflächen-, Form- oder Materialveränderungen) erfährt.

Der Innenraum ist mikrobiell gegen die Umgebung des Containers isoliert. Zur Herbeiführung dieser Isolierung kann der Container beispielsweise geeignete Dichtelemente aufweisen, beispielsweise Gummidichtungen oder Ähnliches, welche ein Eindringen mikrobieller Verunreinigungen in den Container im Prinzip verhindern. Unter "im Prinzip" ist in diesem Zusammenhang eine dem üblichen Standard entsprechende Optimierung der Verhinderung des Eindringens mikrobieller Verunreinigungen zu verstehen, bei welcher über einen praktisch erforderlichen Zeitraum und unter üblichen Bedingungen, welche für den Transfer und die Lagerung der pharmazeutischen Behältnisse benötigt werden (beispielsweise einige Stunden bis hin zu einigen Monaten, unter schwankenden Umgebungsbedingungen (z.B. Temperatur, Luftdruck) sowie unter mechanischer Belastung), keine mikrobiellen Verunreinigungen in den Innenraum eindringen.

Der Innenraum weist mindestens eine Halterung für die Aufnahme mindestens eines Nests zur Lagerung einer Mehrzahl von pharmazeutischen Behältnissen auf. Wie oben beschrieben, kann dieses Nest beispielsweise eine ebene Platte umfassen, welche auch als "Nestkörper" bezeichnet werden kann. Der Nestkörper, welcher im Wesentlichen eben ausgestaltet sein kann, kann dann eine Mehrzahl von Öffnungen zum Einhängen der pharmazeutischen Behältnisse aufweisen. Diese Öffnungen können beispielsweise in einer regelmäßigen Matrix angeordnet sein, in einer Linearanordnung oder in einer hexagonalen, besonders raumsparenden Anordnung. Die Nester können beispielsweise aus einem oder mehreren gegen eine Sterilisation, beispielsweise eine Sterilisation der oben beschriebenen Art, resistenten Material hergestellt sein, beispielsweise entsprechenden Kunststoffen (zum Beispiel fluorierten Kunststoffen, insbesondere Polytetrafluorethylen oder PTFE) und/oder aus Metallen, Keramiken, Verbundwerkstoffen oder Ähnlichem. Besonders bevorzugt ist es, wenn der Container derart ausgestaltet ist, dass dieser eine Vielzahl derartiger Nester aufnehmen kann, so dass eine besonders effiziente Transferierung sterilisierter pharmazeutischer Behältnisse, vorzugsweise in Stückzahlen von mehreren 100 bis hin zu mehreren 1000, erfolgen kann. Die mindestens eine Halterung kann dementsprechend eine auf das mindestens eine Nest angepasste Halterung umfassen, beispielsweise Schienen, Scharniere, Vertiefungen oder Ähnliches, was eine sichere (beispielsweise im Wesentlichen erschütterungsfreie und gegen Verrutschen gesicherte) Transferierung der Nester bzw. pharmazeutischen Behältnisse gewährleistet. Insbesondere sollen die Halterungen derart ausgestaltet sein, dass die Nester aus diesen herausnehmbar (beispielsweise einschiebbar und/oder ausziehbar) gelagert sind.

Der Container weist weiterhin mindestens eine Containertür zum Herausnehmen und/oder Einführen des Nests in den Innenraum auf. Diese Containertür kann grundsätzlich auf eine Vielzahl bekannter Arten ausgestaltet sein, beispielsweise als Klapptür, Schiebetür oder Ähnliches. Dabei ist die Tür derart ausgestaltet, beispielsweise mittels geeigneter Dichtelemente, dass nach wie vor der Innenraum bei geschlossener Tür mikrobiell gegen die Umgebung des Containers isoliert ist. Besonders bevorzugt ist es, wenn diese Containertür auf der Oberseite und/oder, was bevorzugt ist, an einer Seitenwand des Containers (beispielsweise einer Stirnseite eines quaderförmigen Containers) angeordnet ist. Diese Containertür kann insbesondere eine Schwenktür, insbesondere eine sich nach außen öffnende Schwenktür, insbesondere ein Schott, umfassen.

Zusätzlich umfasst der Container mindestens eine Schleuse zum Ankoppeln des Containers an einen Sterilraum. Unter einer "Schleuse" ist dabei ein beliebiger Kopplungsmechanismus zu verstehen, welcher ein mechanisch stabiles Verbinden des Containers mit einem Sterilraum ermöglicht. Als Beispiel seien diesbezüglich Bajonettverschlüsse genannt, wobei jedoch auch andere Arten von Schleusen möglich sind, beispielsweise Schleusen mit entsprechenden Schleusenhaken, Schieber, Klappen oder Ähnliches. Derartige Schleusen sind dem Fachmann grundsätzlich aus anderen Bereichen der Technik bekannt. Erfindungsgemäß wird die Schleuse des Containers jedoch derart ausgestaltet, dass nach dem Ankoppeln an den Sterilraum ein Öffnen der Containertür zumindest ohne eine wesentliche wesentliche Unterbrechung der Sterilität ermöglicht wird. Dieses Öffnen der Containertür kann insbesondere vom Sterilraum aus erfolgen. Unter "zumindest ohne eine wesentliche Unterbrechung der Sterilität" ist dabei insbesondere zu verstehen, dass die Schleuse im angekoppelten Zustand die Containertür gegen einen Außenbereich, welcher den (sich außerhalb des Sterilraums befindenden) Container umgibt, mikrobiell abgeschirmt ist. Zu diesem Zweck kann die Schleuse beispielsweise wiederum mindestens eine entsprechende Dichtung umfassen, beispielsweise eine Dichtung unter Verwendung eines Metallrings oder Metallrahmens, eines Kunststoffrings oder Kunststoffrahmens, oder eine Dichtung unter Verwendung eines Gummirings oder Gummirahmens. Auch andere Arten von Dichtungen sind dabei prinzipiell möglich. Unter "zumindest ohne eine wesentliche Unterbrechung der Sterilität" ist dabei selbstverständlich nicht auszuschließen, dass in dem (vorzugsweise geringen) Volumen, welches sich nach dem Ankoppeln des Containers an den Sterilraum zwischen der Containertür und dem Sterilraum ausbildet, aufgenommene Mikroorganismen enthalten sein können. Dieses Volumen kann gegebenenfalls vor dem Öffnen der Containertür zusätzlich sterilisiert werden und/oder beispielsweise durch Evakuieren mittels einer geeigneten Evakuierungsvorrichtung entleert werden. Auf diese Weise könnte optional die Sterilität bei dem Transfer zwischen dem Container und dem Sterilraum sichergestellt werden.

Der beschriebene Container ist somit derart einsetzbar, dass der Transfer von pharmazeutischen Behältnissen in den Sterilraum vorzugsweise ohne Trays und mehrfache Schutzfolien auskommt. Die pharmazeutischen Behältnisse können somit beispielsweise beim Lieferanten gereinigt werden und je nach Erfordernis beispielsweise vorbehandelt (zum Beispiel an der Spritzenspitze durch eine Kappe verschlossen) werden. Die pharmazeutischen Behältnisse können, nach dem Einbringen in die Nester oder vorher, noch zusätzlich mit dünnen Schutzfolien an ihren Enden verschlossen werden, so dass zusätzlich eine Aufrechterhaltung der Sterilität und/oder eine Sicherheit gegenüber dem Eindringen von Schwebeteilchen/ Partikeln aller Art gewährleistet werden kann.

Die pharmazeutischen Behältnisse, insbesondere die Spritzenkörper, können vor dem Einbringen in den Container gereinigt und/oder sterilisiert werden oder nach dem Einbringen im Innenraum des Containers. Vor dem Einbringen des Inhaltes kann auch der Innenraum als solcher gereinigt und/oder sterilisiert werden.

Der Innenraum des Containers umfasst, abhängig von seiner Größe und den pharmazeutischen Behältnissen, vorzugsweise einige hundert oder mehr pharmazeutische Behältnisse und ersetzt somit in der Regel viele Trays. Der verschlossene, in seinem Inneren samt Inhalt sterilisierte Container kann, beispielsweise vom Hersteller der Behältnisse, hin zum Arzneimittelhersteller bzw. zum Abfüllbetrieb zur Befüllung mit dem pharmazeutischen Medium verbracht werden. Beim Arzneimittelhersteller bzw. Abfüllbetrieb wird der Container beispielsweise zu einem Sterilraum verbracht und dort an eine Schleuse (im Folgenden "Sterilschleuse") des Sterilraums angekoppelt. Danach wird der Container, beispielsweise automatisch, halbautomatisch oder manuell geöffnet, und sein Innenraum samt Inhalt zum Sterilraum hin exponiert, derart, dass die Sterilität des Inneren des Containers samt Inhalt zumindest weitgehend gewährleistet bleibt.

Der Inhalt des Containers, d. h. die Nester und die pharmazeutischen Behältnisse, können auf unterschiedliche Weise entnommen werden, beispielsweise indem die pharmazeutischen Behältnisse aus den Nestern entnommen werden, wobei die Nester ganz oder teilweise im Innenraum des Containers verbleiben. Alternativ können die Nester auch mitsamt den pharmazeutischen Behältnissen aus dem Container oder einem Innencontainer desselben entnommen werden. Weiterhin können die Nester mitsamt den pharmazeutischen Behältnissen auch aus dem Container oder einem Innencontainer herausgefahren, jedoch nicht vollständig entnommen werden. In einer weiteren Alternative enthält der Container einen Innencontainer, in dessen Inneren die Halterungen zur Aufnahme des mindestens einen Nestes aufgenommen sind. Die Containertür sollte dann derartig ausgestaltet sein, dass der Innencontainer reversibel aus dem Innenraum des Containers herausnehmbar ist. In diesem Fall kann der Innenbehälter samt den Nestern und den darin enthaltenen pharmazeutischen Behältnissen aus dem Container entnommen und beispielsweise in den Sterilraum verbracht werden.

Werden aus dem Container oder dessen Innencontainer die Nester samt den leeren pharmazeutischen Behältnissen entnommen, so können erstere an bzw. auf eine Abfülllinie verbracht werden. Mittels geeigneter Formteile kann erreicht werden, dass die Nester auf einer üblichen sogenannten Line-Filler-Abfüllmaschine prozessiert werden können. Line-Filler-Abfüllmaschinen stellen eine gängige pharmazeutische Herstelltechnologie dar. Eine eigene Tray-Filler-Abfüllmaschine wird somit in der Regel nicht benötigt, ist grundsätzlich aber auch einsetzbar.

Die pharmazeutischen Behältnisse können, gegebenenfalls nach Entfernung einer zusätzlichen Versiegelung (wie beispielsweise einer dünnen Schutzfolie), welche den Spritzenkörper auf der Seite der Befüllung gegen ein Eindringen von Partikeln oder anderen Verunreinigungen verschließen kann, befüllt und dann mittels gängiger Technologie beispielsweise mit Stopfen oder anderen Verschlüssen verschlossen. Die derart befüllten, verschlossenen pharmazeutischen Behältnisse werden dann beispielsweise aus den Nestern mittels gängiger "De-Nesting-Technik" entnommen.

Alternativ zu einer Zuführung von Nestern an oder auf die Abfülllinie können die leeren pharmazeutischen Behältnisse vor Eintritt in die Abfülllinie aus den Nestern entnommen und einzeln oder in Gruppen auf die Abfülllinie gesetzt werden. Diese Alternative ist besonders dann vorteilhaft, wenn die pharmazeutischen Behältnisse in den Nestern in einer Geometrie angeordnet sind, welche es nicht erlaubt, die Nester samt Spritzenkörpern ohne zusätzlichen technischen Aufwand auf einer herkömmlichen Abfülllinie zu prozessieren.

Leere Nester können dann in einen Container bzw. einen Innenbehälter zurückverbracht werden, wobei letzterer dann beispielsweise wieder in einen Container eingeführt wird. Hierzu kann entweder derselbe Container und/oder Innenbehälter dienen, aus welchem die Nester zuvor entnommen wurden, oder aber ein zweiter Container mit einem Innenbehälter. Die Anforderungen hinsichtlich der Sterilität sind diesbezüglich nicht mehr so hoch wie bei der Anlieferung der unbefüllten pharmazeutischen Behältnisse. Der mit leeren Nestern bestückte Container mit oder ohne Innenbehälter wird dann vorzugsweise unter Wahrung der aseptischen Reinraumbedingungen in geeigneter Weise aus dem Sterilraum ausgeschleust bzw. von dessen Schleuse abgedockt. Danach kann dieser zurück zum Hersteller der pharmazeutischen Behältnisse transportiert und dort (gegebenenfalls nach Reinigung) erneut mit leeren pharmazeutischen Behältnissen bestückt werden.

Bei der vorgeschlagenen Technologie entstehen somit als unmittelbarer Abfall lediglich gegebenenfalls die dünnen Schutzfolien zum vorübergehenden Schutz der offenen leeren pharmazeutischen Behältnisse.

Der oben beschriebene Container kann auf verschiedene Weisen vorteilhaft ausgestaltet werden, wobei die im Nachfolgenden beschriebenen vorteilhaften Weiterbildungen einzeln oder in Kombination realisiert sein können.

Eine erste bevorzugte Ausgestaltung betrifft die Schleuse selbst. So kann die Schleuse beispielsweise mindestens ein erstes, mit der Außenhülle außerhalb der Containertür verbundenes Schleusenteil umfassen, welches die Containertür zumindest teilweise umgibt. Mittels dieses ersten Schleusenteils kann das Ankoppeln an die Sterilschleuse des Sterilraums, insbesondere ein erstes Sterilschleusenteil, welches eine Sterilraumtür umgibt, angekoppelt werden. Weiterhin kann der Container mindestens ein zweites Schleusenteil umfassen, welches mit der Containertür verbunden ist. Dieses zweite Schleusenteil kann an ein zweites, mit der Sterilraumtür verbundenes Sterilschleusenteil gekoppelt sein. Derartiger doppelte Kopplungen sind beispielsweise aus dem Bereich der Nukleartechnik bekannt und werden dort beispielsweise als "La Calhène"-Schleusen bezeichnet. Derartige La Calhene-Schleusen bewirken, dass besonders effizient und ohne Unterbrechung der Sterilität eine Ankopplung erfolgen kann, da beispielsweise gleichzeitig die ersten Schleusenteile und die zweiten Schleusenteile jeweils mit ihren Gegenstücken verbunden werden. Die Sterilraumtür und die Containertür können durch die Kopplung der mit ihnen jeweils verbundenen zweiten Schleusenteile gleichzeitig geöffnet werden.

Weitere vorteilhafte Ausgestaltungen des Containers ergeben sich hinsichtlich dessen Innraums. So kann beispielsweise der mindestens eine Innenraum, wie oben beschrieben, einen in dem Innenraum aufnehmbaren Innencontainer umfassen. Dieser Innencontainer ist vorzugsweise als herausnehmbarer Innencontainer ausgestaltet, zu welchem Zweck beispielsweise Vorrichtungen zur reversiblen Aufnahme des Innenbehälters vorgesehen sein können. Wie auch der Außencontainer kann auch der Innencontainer beispielsweise von rechteckiger, polygonaler, runder oder ähnlicher geometrischer Form sein oder mit entsprechenden Rundungen ausgestaltet sein. Bezüglich der verwendbaren Materialien kann auf die oben beschriebenen Materialien verwiesen werden, so dass, wie auch für den Außencontainer, auch für den Innencontainer beispielsweise Metalle (zum Beispiel Edelstahl), Keramik, Glas, Kunststoffe (insbesondere fluorierte Kunststoffe, wie beispielsweise Polytetrafluorethylen) und/oder Verbundstoffe derartiger Materialien eingesetzt werden können. Insbesondere können die Materialien derart gewählt sein, wie auch die Materialien des Außencontainers, dass diese den bei Benutzung, Transport und Lagerung auftretenden mechanischen Belastungen sowie vielfachen Reinigungs- und/oder Sterilisationszyklen standhalten können. Insbesondere können die Materialien und die Konstruktion des Containers bzw. des Innencontainers derart ausgestaltet sein, dass diese die mikrobielle Reinheit des Containerinneren gewährleisten. Zu diesem Zweck können die Materialien beispielsweise auch Zusatzstoffe enthalten, welche eine mikrobielle Desinfektion bewirken, wie dies beispielsweise aus Kühlschränken bekannt ist. So können beispielsweise antibakterielle Substanzen verwendet werden, welche beispielsweise in und/oder auf Kunststoffoder Metalloberflächen angelagert werden können.

Um eine verbesserte Sterilisierung, insbesondere durch fluide Medien (wie zum Beispiel die oben beschriebenen Gase, Dämpfe oder Flüssigkeiten) zu gewährleisten, kann der Innencontainer als offener Innencontainer ausgestaltet sein, insbesondere als von fluiden Sterilisationsmedien durchströmbarer Innencontainer. Zu diesem Zweck kann der Innencontainer beispielsweise zwar eine entsprechende mechanische Stabilität aufweisen, jedoch beispielsweise entsprechende Öffnungen aufweisen, welche den Eintritt und/oder das Durchströmen bzw. Beaufschlagen des Innenraums mit Sterilisationsmedien ermöglichen.

Die Sterilisation der pharmazeutischen Behältnisse kann beispielsweise beim Hersteller der Behältnisse erfolgen, beispielsweise in einem zweiten Sterilraum. Alternativ oder zusätzlich kann die Sterilisation jedoch auch unmittelbar innerhalb des Containers durchgeführt werden, beispielsweise mittels der oben beschriebenen Sterilisationsmedien. Zu diesem Zweck kann insbesondere der Container, beispielsweise der Außencontainer, mindestens eine Verbindungsöffnung zum Einleiten und/oder Ausleiten mindestens einen fluiden Desinfektions- oder Sterilisationsmediums in den bzw. aus dem Innenraum aufweisen. Die Verbindungsöffnung kann insbesondere verschließbar ausgestaltet sein, beispielsweise durch ein entsprechendes Ventil, und kann insbesondere mindestens einen Stutzen (zum Beispiel einen Stutzen mit einem standardisierten Flansch) umfassen. Auf diese Weise können entsprechende Sterilisationsmedien (zum Beispiel Gase oder Heißdampf) zur Reinigung und/oder Sterilisation des Containerinnenraums eingeleitet und wieder aus diesem ausgeleitet werden.

Um eine verbesserte Durchmischung der Atmosphäre im Containerinnenraum zu ermöglichen, beispielsweise während einer Sterilisation und/oder später während eines Transports, können zusätzlich ein oder mehrere Ventilationsvorrichtungen im Inneren des Containers vorgesehen sein.

Weiterhin kann, ebenfalls um eine Sterilisation bzw. Desinfektion zu bewirken, der Container mindestens eine Öffnung, vorzugsweise wiederum eine verschließbare Öffnung (beispielsweise wiederum mittels eines geeigneten Ventils) zum Einbringen einer Strahlungsquelle in den Innenraum aufweisen. Wie oben beschrieben, kann diese Strahlungsquelle beispielsweise eine intensive Lichtquelle (zum Beispiel eine ultraviolette Lichtquelle), eine anders gestaltete, elektromagnetische Strahlenquelle, eine radioaktive Strahlenquelle (zum Beispiel eine Alpha-, Beta- oder Gamma-Quelle oder eine Kombination derartiger Strahlenquellen umfassen. Der Container kann derart ausgestaltet sein, dass diese Strahlenquelle nur vorübergehend über die Öffnung in diesem Container eingebracht werden kann, oder der Container kann vollständig und integral eine derartige Strahlenquelle zur Desinfektion und/oder Sterilisation des Innenraums bzw. der pharmazeutischen Behältnisse umfassen. Alternativ oder zusätzlich zu den bereits genannten Möglichkeiten ist es weiterhin möglich, den Container mit mindestens einer Temperiervorrichtung zum Heizen und/oder Kühlen des Innenraums auszugestalten. Beispielsweise können hierfür entsprechende Heizstrahler und/oder anders gestaltete Heizeinrichtungen vorgesehen sein, wobei das Aufheizen beispielsweise bis zu einer thermischen Desinfektion durchgeführt werden kann.

Weiterhin kann der Container bzw. dessen Innenraum vollständig gegen den Außenbereich isoliert sein, beispielsweise durch eine druckdichte und/oder vakuumdichte Abschottung. Wie oben dargelegt, ist es jedoch im Wesentlichen nur erforderlich, eine mikrobielle Abschirmung zu gewährleisten. Zu diesem Zweck kann beispielsweise der Container weiterhin mindestens einen mikrobiellen Filter zur Filterung von in dem Innenraum umgewälzter Atmosphäre und/oder zur Filterung von in den Innenraum eindringenden fluiden Medien, insbesondere Luft, umfassen.

Der beschriebene Container in einer der beschriebenen Ausgestaltungen soll insbesondere für einen Transport, beispielsweise von einem Hersteller der pharmazeutischen Behältnisse hin zu einem Medikamentenhersteller und/oder Abfüllbetrieb, geeignet sein. Zu diesem Zweck kann der Container beispielsweise eine Mehrzahl von Rollen, insbesondere Rollen mit Bremseinrichtungen, umfassen. Unter "Rollen" sind dabei grundsätzlich beliebige Arten von Vorrichtungen zur Erleichterung der Fortbewegung zu verstehen, also neben Rollen im eigentlichen Sinn auch Räder, Zahnräder, Kufen, Schienen oder ähnliches. Daneben, also insbesondere neben einer Mehrzahl von Rollen, kann optional der Container auch eine Mehrzahl von Lagerfüßen zur stationären Lagerung des Containers umfassen, beispielsweise in Form von nicht-rollenden Füßen zur feststehenden Lagerung des Containers. Auf diese Weise können während der Lagerung schwerer Container die Containerrollen entlastet und zum Beispiel ein unbeabsichtigtes Wegrollen vermieden werden. Die Lagerfüße können beispielsweise als ausfahrbare Lagerfüße ausgestaltet sein, welche während einer stationären Lagerung in Bodenkontakt gebracht werden.

Besonders bevorzugt ist es, wenn eine Vielzahl der beschriebenen Container einzeln oder im Verband transportiert werden kann. Zu diesem Zweck kann der vorgeschlagene Container beispielsweise äußere Formprofile aufweisen, welche derart ausgestaltet sind, dass eine Mehrzahl von Containern stapelbar ist. Beispielsweise können dementsprechend die Container entsprechende Vertiefungen aufweisen, in welche die Lagerfüße und/oder Rollen benachbarter, insbesondere sich nach oben hin anschließender Container aufnehmen können. Auf diese Weise kann ein sicheres Stapeln oder eine sichere Lagerung, ein Transport, eine Fortbewegung und ein Betrieb einzelner oder im Verband befindlicher Container gewährleistet werden. Alternativ oder zusätzlich kann der Container weiterhin mindestens eine von außen zugängliche Kupplung zum Ankoppeln einer Transporteinrichtung, beispielsweise eines Hakens bzw. Kranhakens oder Transporthakens, aufweisen. Derartige Kupplungen können beispielsweise Ösen oder komplexere Kupplungen umfassen.

Wie oben beschrieben, kann der Container einen Innencontainer aufweisen. Im Innenraum des Containers oder gegebenenfalls des Innencontainers können ein oder mehrere horizontal oder vertikal angeordnete Schienen oder vergleichbare geeignete Vorrichtungen zur reversiblen Aufnahme von Nestern vorgesehen sein. In den Nestern können dann die pharmazeutischen Behältnisse, welche als Primärpackmittelkörper dienen können, aufgenommen sein. Der Innenraum des Containers kann weiterhin, wie oben beschrieben, Vorrichtungen zur reversiblen Aufnahme des herausnehmbaren Innencontainers aufweisen. Der Innencontainer kann prinzipiell aus den gleichen Materialien gefertigt sein wie beispielsweise die Außenhülle des Containers, wobei jedoch auch eine Verwendung unterschiedlicher Materialien, entsprechend unterschiedlichen Anforderungen, möglich ist. Die beschriebenen Ausgestaltungen mit der Verbindungsöffnung, der Ventilationsvorrichtung, der Öffnung zum Einbringen der Strahlungsquelle, der Strahlungsquelle selbst, der Temperiervorrichtung, des Filters, und die weiteren bezüglich des gesamten Containers beschriebenen Ausgestaltungen können insbesondere auch speziell auf den Innencontainer angewandt werden. Der Innencontainer kann insbesondere Schienen oder andere geeignete Vorrichtungen zur Aufnahme der Nester aufweisen. Der Innencontainer, der äußere Container und die Nester sollen insbesondere derart ausgelegt sein, dass diese mehrere bis viele Zyklen von Reinigung, Beladung mit pharmazeutischen Behältnissen, Sterilisation, Transport zum Arzneimittelhersteller bzw. Abfüller, Entladung und Rücktransport gewährleisten. Auf diese Weise ermöglicht es die beschriebene Technologie, unterschiedliche Abfüllsysteme (zum Beispiel Line-Filler, Tray-Filler) mit abfüllfertigen Primärbehältnissen zu beliefern, wodurch eine hohe Flexibilität der beschriebenen Container möglich ist.

Neben den Containern in einer oder mehreren der beschriebenen Ausgestaltungen wird weiterhin ein Transfersystem für den sterilen Transfer pharmazeutischer Behältnisse in einen Sterilraum vorgeschlagen. Das Transfersystem umfasst mindestens einen Container nach einer oder mehreren der oben beschriebenen Ausgestaltungen, sowie weiterhin mindestens einen Sterilraum. Unter einem "Sterilraum" ist dabei ein zumindest teilweise abgeschlossener bzw. abgeschirmter Raum zu verstehen, in welchem eine erhöhte Keimfreiheit gegenüber einem Außenraum herrscht. Dies kann beispielsweise durch entsprechende Schleusen, mikrobielle Filter, Sterilisationseinrichtungen oder Ähnliches bewirkt werden. Der Sterilraum kann insbesondere als begehbarer Sterilraum ausgestaltet sein. Der Sterilraum weist mindestens eine Sterilraumwand mit einer Sterilschleuse auf, welche an die Schleuse des Containers ankoppelbar ist. Der Sterilraum kann beispielsweise mindestens eine Abfülllinie zum Abfüllen mindestens eines pharmazeutischen Mediums in die pharmazeutischen Behältnisse aufweisen.

Neben dem Container und dem Transfersystem in einer der beschriebenen Ausgestaltungen wird weiterhin erfindungsgemäß ein Verfahren zum Befüllen pharmazeutischer Behältnisse mit mindestens einem pharmazeutischen Medium vorgeschlagen. Das Verfahren kann insbesondere zur Befüllung von Spritzenkörpern eingesetzt werden, wobei jedoch auch andere Füllmedien und/oder andere Arten von Behältnissen befüllt werden können. Insbesondere kann das Verfahren unter Verwendung mindestens eines Containers nach einem oder mehreren der oben beschriebenen Ausführungsbeispiele durchgeführt werden, so dass weitgehend auf die obigen Definition und Beschreibungen verwiesen werden kann. Das erfindungsgemäße Verfahren umfasst die nachfolgend beschriebenen Verfahrensschritte. Die Schritte können vorzugsweise in der angegebenen Reihenfolge durchgeführt werden, was jedoch nicht zwingend erforderlich ist. Weiterhin können ein oder mehrere der beschriebenen Verfahrensschritte auch wiederholt durchgeführt wird, sowie zeitlich parallel und/oder zeitlich überlappend. Neben den geschilderten Verfahrensschritten kann das Verfahren auch weitere, nicht genannte Schritte aufweisen.

In einem ersten Verfahrensschritt wird eine Mehrzahl pharmazeutischer Behältnisse hergestellt. Beispielsweise können diese Behältnisse Glasbehältnisse, wie beispielsweise Spritzenkörper, umfassen. Neben Glas können auch andere Materialien eingesetzt werden, beispielsweise Keramik, Kunststoffe, Papier oder Verbundwerkstoffe.

In einem weiteren Verfahrensschritt werden die pharmazeutischen Behältnisse sterilisiert. Diese Sterilisation kann beispielsweise mittels Sterilisationsmedien (zum Beispiel fluiden Sterilisationsmedien, beispielsweise Sterilisationsflüssigkeiten und/oder Gasen und/oder Heißdampf) der oben beschriebenen Art erfolgen. Alternativ oder zusätzlich können auch thermische Sterilisierungsmethoden, Sterilisierung durch radioaktive Strahlung oder Sterilisierung durch andere Arten von Strahlung, insbesondere elektromagnetische Strahlung, beispielsweise UV-Strahlung, durchgeführt werden, sowie allgemein Dampfsterilisationen. Wie oben dargelegt, kann diese Sterilisation unmittelbar beim Hersteller der pharmazeutischen Behältnisse erfolgen und/oder nach einem Einbringen der pharmazeutischen Behältnisse in ein Nest und/oder einen Container.

In einem weiteren Verfahrensschritt werden die pharmazeutischen Behältnisse in mindestens ein Nest zur Lagerung einer Mehrzahl von pharmazeutischen Behältnissen eingebracht. Dieses Nest wird in einen wieder verwendbaren Container eingebracht, welcher eine Außenhülle mit einem sterilisierbaren Innenraum und eine Containertür umfasst. Der Innenraum ist mikrobiell gegen die Umgebung des Containers isoliert und weist mindestens eine Halterung für die Aufnahme des mindestens einen Nestes, vorzugsweise für die Aufnahme einer Vielzahl von Nestern, auf. Wie oben beschrieben, kann die Sterilisation auch nach der Aufnahme der Nester mit den pharmazeutischen Behältnissen in dem Innenraum erfolgen.

Anschließend wird in einem weiteren Verfahrensschritt der derart befüllte Container zu einem Sterilraum verbracht und über mindestens eine Schleuse an den Sterilraum angekoppelt. Bezüglich der Ausgestaltung des Sterilraums kann beispielsweise auf die obigen Ausführungen verwiesen werden. Dabei ist die Schleuse derart ausgestaltet, dass nach dem Ankoppeln an den Sterilraum ein Öffnen der Tür vom Sterilraum zumindest ohne wesentliche Unterbrechung der Sterilität ermöglicht wird.

Nach diesem Ankoppeln wird das Nest durch die Containertür in den Sterilraum überführt. Dort kann dann das mindestens eine pharmazeutische Medium in die Behältnisse eingebracht werden. Dabei kann beispielsweise eine Line-Filler-Anlage verwendet werden, bei welcher die pharmazeutischen Behältnisse nacheinander befüllt werden. Alternativ oder zusätzlich kann auch eine sogenannte Tray-Filler-Anlage verwendet werden, bei welcher sämtliche in dem Nest befindlichen pharmazeutischen Behältnisse oder eine Mehrzahl dieser Behältnisse gleichzeitig befüllt werden.

Nach dem Befüllen kann, wie oben beschrieben, der Container entweder leer oder zumindest mit leeren Nestern beladen wieder von dem Sterilraum entkoppelt werden und zurück, beispielsweise zum Ort der Herstellung der pharmazeutischen Behältnisse, verbracht werden.

### Ausführungsbeispiele

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: eine schematische Schnittdarstellung eines erfindungsgemäßen Containers für den sterilen Transfer von Spritzenkörpern;
- Figur 2A und 2B: zwei Ausführungsbeispiele verschiedener Nester für die Aufnahme von Spritzenkörpern; und
- Figur 3: einen Ausschnitt eines erfindungsgemäßen Transfersystems mit einem an eine Sterilschleuse angekoppelten Container in Schnittdarstellung.

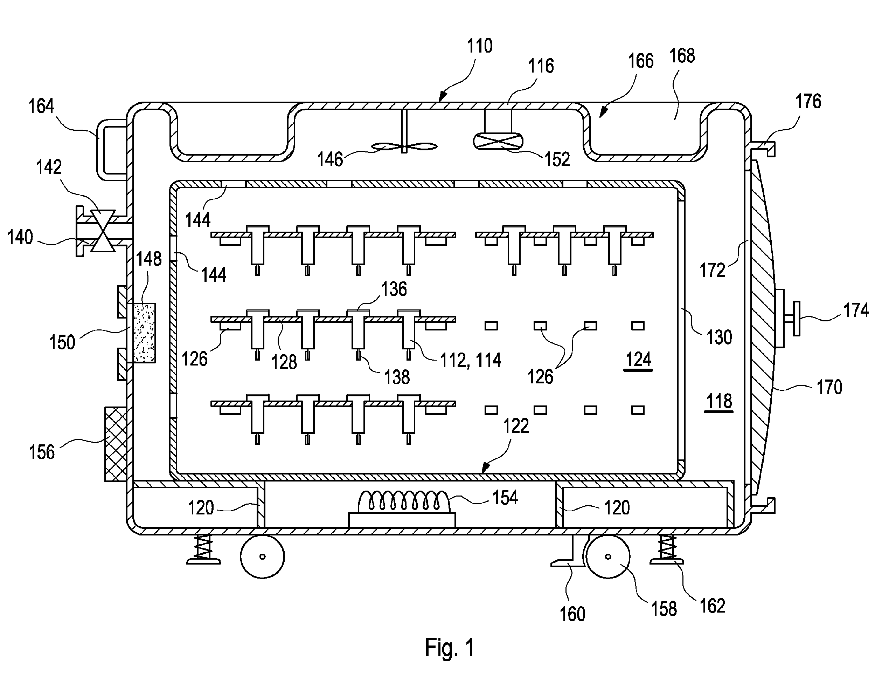

In Figur 1 ist in stark schematisierter Darstellung ein Ausführungsbeispiel eines erfindungsgemäßen, wiederverwendbaren Containers 110 für den sterilen Transfer pharmazeutischer Behältnisse 112 in Schnittdarstellung von der Seite gezeigt. Die pharmazeutischen Behältnisse 112 sind in diesem Ausführungsbeispiel ohne Beschränkung der Möglichkeit der Verwendung weiterer Arten von Behältern als Spritzenkörper 114 ausgestaltet.

Der Container 110 weist eine Außenhülle 116 auf, welche in diesem Ausführungsbeispiel als einwandige Außenhülle 116 ausgestaltet ist. Es sind jedoch auch beispielsweise doppeloder mehrwandige Ausgestaltungen möglich. Die Außenhülle kann beispielsweise aus einem oder mehreren der oben erwähnten Materialien hergestellt sein und soll insbesondere eine Formstabilität gewährleisten, um den mechanischen Belastungen beim Transport (beispielsweise einem Stapeln mehrerer Container 110) standzuhalten. Weiterhin soll die Außenhülle 116 intensiven Reinigungs- und gegebenenfalls Desinfektions- bzw. Sterilisationsprozessen standhalten können.

Die Außenhülle 116 umschließt einen sterilisierbaren Innenraum 118. In diesem Innenraum 118 sind Vorrichtungen 120 zur Aufnahme eines Innencontainers 122 aufgenommen. Diese Vorrichtungen 120 können beispielsweise Schienen, Schwalbenschwanzführungen, Feststellvorrichtungen, Gewinde, Andockstationen oder Ähnliches umfassen.

Der Innencontainer 122 ist in diesem einfachen Ausführungsbeispiel gemäß Figur 1 lediglich stark schematisiert dargestellt. Dabei ist der Innencontainer 122 in diesem Ausführungsbeispiel als einfacher, quaderförmiger Container ausgestaltet und ist beispielsweise ebenfalls aus den für den Container 110 infrage kommenden Materialien hergestellt. Auch der Innencontainer 122 soll eine Formstabilität, auch bei Beladung mit pharmazeutischen Behältnissen 112, gewährleisten. Beispielsweise kann der Innencontainer 122 wiederum aus Edelstahl gefertigt sein.

Der Innencontainer 122 ist somit im Innenraum 118 des Containers 110 aufgenommen. Der Innencontainer 122 weist seinerseits einen Innenraum 124 auf, in welchem Halterungen 126 für die Aufnahme von Nestern 128 angeordnet sind. Diese Haltungen 126 können wiederum verschiedene Arten von Halterungen umfassen, beispielsweise Schienen zum Einschieben der Nester 128 durch eine Öffnung 130 zum Beladen des Innencontainers 122.

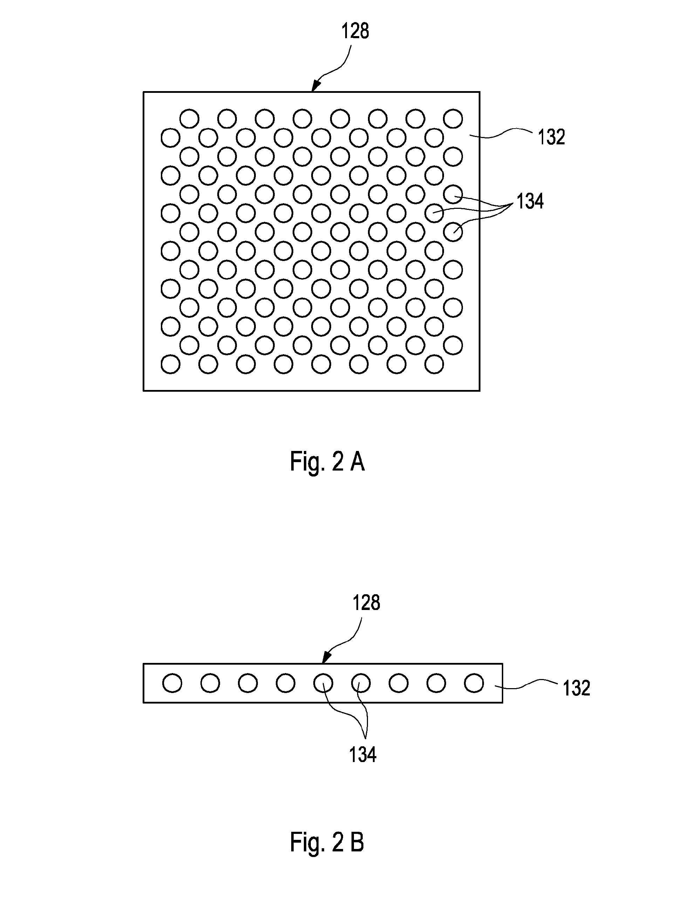

Die Nester 128 dienen zur Aufnahme der Spritzenkörper 114. Ausführungsbeispiele derartiger Nester 128 sind in den Figuren 2A und 2B in Draufsicht gezeigt. Die Nester 128 weisen jeweils einen Nestkörper 132 von flacher Gestalt auf. Der Nestkörper 132 kann als wiederverwendbarer Nestkörper ausgestaltet sein und kann dementsprechend ein formstabiles, auch gegen mehrfache Reinigungs- und Desinfektionsprozesse resistentes Material umfassen, beispielsweise einen Kunststoff (zum Beispiel einen fluorierten Kunststoff) und/oder ein Metall, eine Keramik oder Ähnliches. Jeder Nestkörper 132 weist eine Vielzahl von Öffnungen 134 zur Aufnahme der Spritzenkörper 114 auf. Diese Öffnungen 134 sind in den dargestellten Ausführungsbeispielen als runde Bohrungen im Nestkörper 132 ausgestaltet. Dabei stellt Figur 2A ein Ausführungsbeispiel eines Nestes 128 dar, bei welchem einhundertzwölf Öffnungen 134 in einer im Wesentlichen rechteckförmigen Matrix mit hexagonaler Verteilung angeordnet sind. Jede zweite Reihe ist dabei um einen halben Öffnungsabstand gegenüber der vorhergehenden Reihe verschoben. Auf diese Weise lässt sich eine optimale Raumausnutzung des Nestes 128 gewährleisten.

Figur 2B zeigt hingegen ein Nest 128, bei welchem die Öffnungen 134 in einer Reihe angeordnet sind.

Die in den Figuren 2A und 2B gezeigten Ausführungsbeispiele stellen nur zwei einer Vielzahl von möglichen Ausführungsbeispielen von Nestern 128 dar. Jeder Container 110 bzw. jeder Innencontainer 122 nimmt vorzugsweise eine Vielzahl derartiger Nester 128 auf, so dass beispielsweise jeder Container 110 mit mehreren 100 bis über 1000 Spritzenkörpern 114 befüllt werden kann. Die Form der Nester 128 kann insbesondere auch auf die zu beschickende Abfüllanlage angepasst sein, wobei beispielsweise die Reihenanordnung gemäß Figur 2B für serielle Anlagen (vorzugsweise sog. "Line Filler") geeignet sein kann, die Anordnung gemäß Figur 2A für Anlagen mit einer zweidimensionalen Positionierung der zu befüllenden Spritzenkörper 114. Dabei können die Anlagen beispielsweise derart ausgestaltet sein, dass die Spritzenkörper 114 vor Befüllung aus den Nestern 128 entnommen werden, oder es kann, alternativ oder zusätzlich, auch eine Befüllung in den Nestern 128 erfolgen. Weiterhin kann auch die Form der Öffnungen 130 auf die Art der pharmazeutischen Behältnisse 112 bzw. auf die Besonderheiten der Abfüllanlage eingerichtet sein, so dass anstelle von durchgehenden Bohrungen beispielsweise auch Nuten im Rand des Nestkörpers 132 gemäß Figur 2B vorgesehen sein können, in welche die Spritzenkörper 114 seitlich eingehängt werden können. Dies kann unter Umständen eine Entnahme erleichtern. Eine Vielzahl möglicher Ausgestaltungen ist denkbar. Insbesondere können auch die Halterungen 126 auf die besondere Form der Nestkörper 132 angepasst sein.

Wie in Figur 1 teilweise und schematisch gezeigt, können die Spritzenkörper 114 bzw. pharmazeutischen Behältnisse 112, welche in die Öffnungen 134 eingesteckt sind, an ihrem oberen Ende mit einer zusätzlichen Schutzfolie 136 verschlossen sein. Dies ist jedoch nicht zwingend erforderlich. Weiterhin können die Spritzenkörper 114 an ihrem unteren Ende mit einer Kappe 138 verschlossen sein, was in Figur 1 ebenfalls symbolisch angedeutet ist.

Die pharmazeutischen Behältnisse 112 bzw. die Spritzenkörper 114 können beispielsweise unmittelbar beim Hersteller oder einem weiteren Betrieb bzw. Betriebsteil nach der Herstellung gereinigt und desinfiziert bzw. sterilisiert werden. Alternativ oder zusätzlich kann die Desinfektion bzw. Sterilisation auch ganz oder teilweise im Innenraum 118 des Containers 110 erfolgen, bzw. ein sterilisierter bzw. desinfizierter Zustand kann im Innenraum 118 des Containers 110 aufrechterhalten werden. Zu diesem Zweck weist der Container 110 verschiedene Einrichtungen auf, welche eine derartige Desinfektion bzw. Sterilisierung begünstigen oder bewirken können. Diese, allesamt optionalen und beliebig kombinierbaren Einrichtungen können verschiedene Ausführungen umfassen.

So weist der in Figur 1 dargestellte Container 110 beispielsweise einen Stutzen 140 an seiner Rückwand auf. Dieser Stutzen 140, welcher in diesem Ausführungsbeispiel beispielsweise mit einem Ventil 142 zum Verschließen des Stutzens 140 versehen ist, kann insbesondere zum Einleiten und/oder Ausleiten mindestens eines fluiden Desinfektionsmediums und/oder Sterilisationsmediums in den Innenraum 118 dienen. Es kann eine zusätzliche Öffnung vorgesehen sein, so dass dieses Sterilisationsmedium durch den Innenraum 118 hindurchgeleitet werden kann. Auch der Innencontainer 122 kann mit einem entsprechenden Stutzen und/oder einer Öffnung versehen sein, welche ein Einleiten des Sterilisationsmediums (zum Beispiel Ethylenoxid) ermöglicht. In dem in Figur 1 gezeigten Ausführungsbeispiel weist der Innencontainer 122 jedoch eine einfachere Ausgestaltung mit einer Mehrzahl von Öffnungen 144 an seiner Rückwand und seiner Oberseite auf. Diese Öffnungen 144 bewirken, dass der Innencontainer 122 von einem fluiden Sterilisationsmedium durchströmbar ist.

Weiterhin weist in diesem Ausführungsbeispiel der Container 110 eine Ventilationsvorrichtung 146 auf. Diese Ventilationsvorrichtung 146 kann, beispielsweise während des Transports und/oder während eines Sterilisationsprozesses, für eine Durchmischung der Innenraumatmosphäre im Innenraum 118 des Containers 110 bzw. im Innenraum 124 des Innencontainers 122 sorgen. In dem in Figur 1 dargestellten Ausführungsbeispiel ist die Ventilationsvorrichtung 146 lediglich an der Außenhülle 116 vorgesehen. Es können jedoch, alternativ oder zusätzlich, weitere Ventilationsvorrichtungen 146 vorgesehen sein, beispielsweise im Innencontainer 122.

Weiterhin weist der Container 110 optional bei dem in Figur 1 dargestellten Ausführungsbeispiel einen mikrobiellen Filter 148 auf. In dem in Figur 1 dargestellten Ausführungsbeispiel ist der Filter 148 mit einer Öffnung 150 im Container 110 verbunden. Diese Öffnung 150 kann beispielsweise als verschließbare Öffnung ausgestaltet sein. Der mikrobielle Filter 148 sorgt für eine keimfreie Atmosphäre im Inneren des Containers 110. Alternativ oder zusätzlich zu der in Figur 1 gezeigten Anordnung des optionalen Filters 148 kann auch beispielsweise ein Filter 148 am Innencontainer 122 angeordnet sein. Weiter alternativ oder zusätzlich kann auch ein Filter 148 mit einer Umwälzvorrichtung, beispielsweise der Ventilationsvorrichtung 146, im Innenraum 118 des Containers 110 und/oder im Innenraum 124 des Innencontainers 122 vorgesehen sein, so dass nur umgewälzte Luft bzw. Atmosphäre im Inneren des Containers 110 gefiltert wird und kein Eindringen von Luft aus der Umgebung des Containers 110 ins Innere des Containers 110 möglich ist.

Als weitere optionale Vorrichtungen zur Gewährleistung der Sterilität innerhalb des Containers 110 weist der Container 110 und/oder der Innencontainer 122 in dem in Figur 1 gezeigten Ausführungsbeispiel eine Strahlungsquelle 152 auf. Diese Strahlungsquelle 152, welche beispielsweise eine elektromagnetische und/oder radioaktive Strahlungsquelle 152 umfassen kann, ist in dem in Figur 1 gezeigten Ausführungsbeispiel fest im Container 110 integriert. Alternativ oder zusätzlich können jedoch auch Öffnungen im Container 110 bzw. im Innencontainer 122 vorgesehen sein, welche ein lediglich temporäres Einbringen einer derartigen Strahlungsquelle 152 für einen Desinfektions- bzw. Sterilisationsvorgang ermöglichen.

Als weitere optionale Maßnahme zur Aufrechterhaltung bzw. Gewährleistung der Sterilität im Container 110 ist in dem in Figur 1 gezeigten Ausführungsbeispiel eine Temperiervorrichtung 154 vorgesehen. Diese Temperiervorrichtung 154 ist in dem gezeigten Ausführungsbeispiel an der Außenhülle 116 vorgesehen, kann jedoch alternativ oder zusätzlich auch im Innenraum 124 des Innencontainers 122 vorgesehen sein. Die Temperiervorrichtung 154 ist hier als einfache Heizwendel dargestellt, wobei jedoch auch andere Arten von Temperiervorrichtungen vorgesehen sein können. Die Temperiervorrichtung kann vorzugsweise für eine Erhöhung der Temperatur der Atmosphäre in den Innenräumen 118 und/oder 124 des Containers 110 bzw. des Innencontainers 122 sorgen, um eine Keimfreiheit zu gewährleisten. Alternativ oder zusätzlich sind jedoch auch Kühlvorrichtungen denkbar, beispielsweise um die Ausbreitung und/oder Vermehrung mikrobieller Verunreinigungen zu verhindern. In diesem Fall können die Innenräume 118 bzw. 124 beispielsweise mit einem feuchtigkeitsfreien Inertgas, beispielsweise Stickstoff oder Argon, gefüllt werden, um Kondensationen zu vermeiden.

Weiterhin weist optional der Container 110 in dem in Figur 1 dargestellten Ausführungsbeispiel eine Steuerung 156 auf. Diese Steuerung 156 kann beispielsweise eine eigene Energieversorgung für die genannten Vorrichtungen zur Aufrechterhaltung der Sterilität im Container 110 beinhalten, beispielsweise eine Batterie und/oder einen Akkumulator. Alternativ oder zusätzlich können auch Anschlüsse für eine externe Energieversorgung vorgesehen sein, welche beispielsweise an eine entsprechende Energieversorgung in einem Transportmittel und/oder beim Hersteller der pharmazeutischen Behältnisse 112, 114 angeschlossen werden kann. Alternativ oder zusätzlich kann die Steuerung 156 auch Steuerungselemente zum Betätigen der genannten Vorrichtungen zur Aufrechterhaltung bzw. Gewährleistung der Sterilität im Container 110 beinhalten, beispielsweise Schalter, Mikroprozessoren, Schnittstellen zum Anschluss einer externen Steuerung, Ein- und Ausgabemittel (zum Bespiel ein Display, Tasten, Auswahlelemente etc.). Die Gestaltung der Steuerung 156 kann insbesondere auf die besondere Ausgestaltung der Einrichtungen des Containers 110 abgestimmt sein.

Der Container 110 weist weiterhin eine Mehrzahl von Einrichtungen auf, welche einen einfachen Transport des Containers 110 ermöglichen. So sind insbesondere bei dem in Figur 1 gezeigten Ausführungsbeispiel Rollen 158 vorgesehen, welche mit einer Feststell- und Bremsvorrichtung 160 ausgestattet sein können. Neben den Rollen 158 sind weiterhin Lagerfüße 162 optional vorgesehen, welche während einer stationären Lagerung des Containers 110 ausgefahren werden können (beispielsweise durch ein Gewinde, eine ausziehbare Vorrichtung oder Ähnliches), um eine Entlastung der Rollen 158 und eine Feststellung des Containers 110 zu gewährleisten. Für eine Vereinfachung des Transports kann der Container 110 weiterhin Transportösen 164 umfassen, welche in dem in Figur 1 dargestellten Ausführungsbeispiel 164 als einfache Griffe ausgestaltet sind. Es können jedoch, alternativ oder zusätzlich, eine Vielzahl weiterer Kupplungen zum Ankoppeln einer Transporteinrichtung vorgesehen sein, beispielsweise Kupplungen zum Ankoppeln von Transporthaken etc.

Weiterhin ist der Container 110 in dem in Figur 1 dargestellten Ausführungsbeispiel mit äußeren Formprofilen 166 ausgestattet, welche ein Stapeln mehrerer Container 110 ermöglichen. Diese äußeren Formprofile 166 weisen in dem in Figur 1 dargestellten Ausführungsbeispiel insbesondere Vertiefungen 168 an der Decke des Containers 110 auf, in welche die Rollen 158, die Feststell- und Bremsvorrichtungen 160 und die Lagerfüße 162 eines darüber gestapelten Containers 110 (in Figur 1 nicht dargestellt) aufgenommen werden können. Auf diese Weise kann die Standsicherheit gestapelter Container 110 erhöht werden, und die Container 110 können dichter gepackt werden.

Um die Innenräume 118 bzw. 124 des Containers 110 mikrobiell gegen die Umgebung des Containers 110 zu isolieren, weist der Container 110 zusätzlich eine Containertür 170 auf, welche eine Containeröffnung 172 verschließt. Diese Containeröffnung 172 ist in dem in Figur 1 gezeigten Ausführungsbeispiel an einer Stirnseite des Containers 110 angeordnet und ist derart dimensioniert, dass der Innencontainer 122 durch diese Containeröffnung 172 in den Innenraum 118 des Containers 110 eingeschoben werden kann. Alternativ zu dem in Figur 1 gezeigten Ausführungsbeispiel kann der Container 110 auch mehrere Innencontainer 122 aufnehmen, wobei in diesem Ausführungsbeispiel vorzugsweise mehrere Vorrichtungen 120 zur Aufnahme derartiger Innencontainer 122 vorgesehen sein können.

Die Containertür 170 ist in Figur 1 stark schematisiert dargestellt und weist einen Verschlussmechanismus 174 auf, beispielsweise einfache Drehverschlüsse, Haken etc. Insbesondere kann beispielsweise ein Schott als Containertür 170 verwendet werden.

Die Containertür 170 bzw. die Containeröffnung 172 sind in dem in Figur 1 dargestellten Ausführungsbeispiel von einer Schleuse 176 zum Ankoppeln des Containers an einen Sterilraum umgeben. Diese Schleuse 176 ist derart ausgestaltet, dass im angekoppelten Zustand die Containertür 170 geöffnet werden kann, ohne dass Atmosphäre aus dem den Container 110 umgebenden Bereich in die Innenräume 118 bzw. 124 eindringen kann. Lediglich Atmosphäre aus dem Inneren des Sterilraums bzw. der Schleuse des Sterilraums (in Figur 1 nicht dargestellt) können in den Container 110 bei geöffneter Containertür 170 eindringen.

Für die Ausgestaltung der Schleuse 176 des Containers 110 sind verschiedene Möglichkeiten denkbar, wobei in Figur 1 stark schematisiert die Möglichkeit eines Bajonettverschlusses gezeigt ist. Zum Ankoppeln kann die Schleuse 176 auch bewegliche, beispielsweise drehbare und/oder schwenkbare und/oder flexible (beispielsweise schlauchförmige), Teile umfassen.

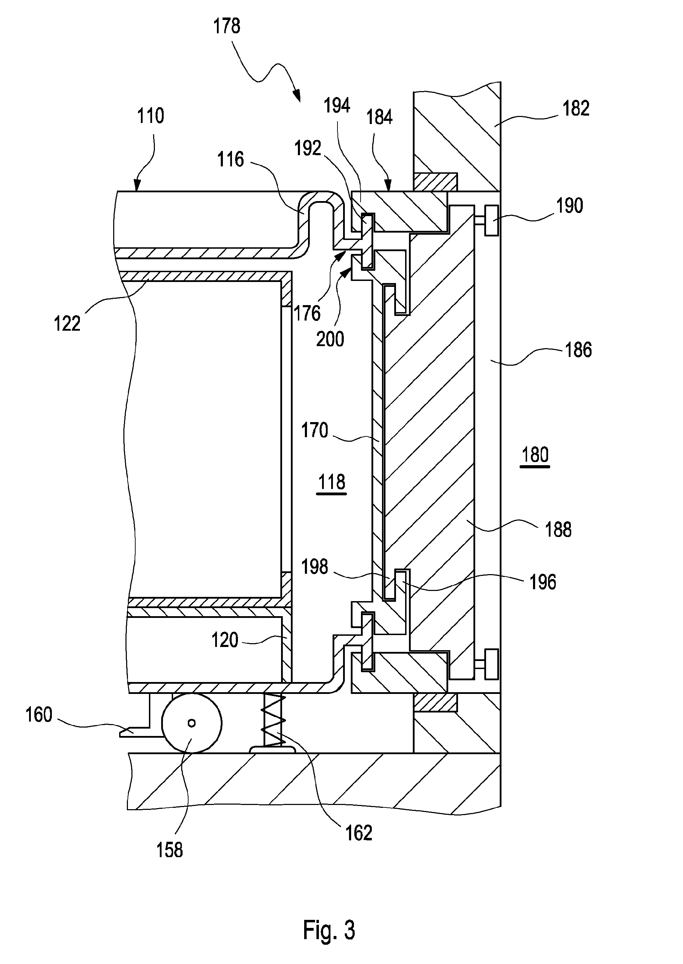

Eine mögliche Ausgestaltung einer Schleuse 176 ist stark schematisiert in Figur 3 in Schnittdarstellung gezeigt. Dabei ist schematisch ein Transfersystem 178 gezeigt, welches einen Container 110, beispielsweise gemäß dem Ausführungsbeispiel in Figur 1, und einen begehbaren Sterilraum 180 umfasst. Der Container 110 ist dabei in der in Figur 3 gezeigten Situation über seine Schleuse 176 an eine mit einer Sterilraumwand 182 gekoppelte Sterilschleuse 184 verbunden. Diese Sterilschleuse 184 kann eine Sterilraumöffnung 186 umgeben, welche insbesondere an die Dimensionen der Containertür 170 angepasst sein kann. Auf diese Weise kann aus dem Sterilraum 180 heraus bei angekoppeltem Container 110 beispielsweise die Containertür 170 geöffnet werden, ohne die sterile Innenatmosphäre im Innenraum 118 des Containers 110 zu unterbrechen.

Die Sterilschleuse 184 umfasst eine Sterilraumtür 188. Diese Sterilraumtür 188 verschließt im nicht-gekoppelten Zustand mittels eines Verriegelungsmechanismus 190, welcher in Figur 3 lediglich stark schematisiert dargestellt ist, die Sterilraumöffnung 186. Die Schleuse 176 des Containers 110 umfasst bei dem in Figur 3 dargestellten Schleusenprinzip ein erstes Schleusenteil 192, welches mit der Außenhülle 116 des Containers 110 außerhalb der Containertür 170 verbunden ist und die Containertür 170 beispielsweise ringförmig umgibt. Dieses erste Schleusenteil 192 ist beispielsweise als umlaufender Vorsprung, gegebenenfalls versehen mit Unterbrechungen, ausgestaltet und erlaubt die Ankopplung an ein erstes Sterilschleusenteil 194. Dieses erste Sterilschleusenteil 194 umgibt im Wesentlichen die Sterilraumöffnung 186 ringförmig und gewährleistet im angekoppelten Zustand eine mikrobielle Isolierung des Sterilraums 180 und des Innenraums 118 des Containers 110. Das erste Schleusenteil 192 und das erste Sterilschleusenteil 194 können beispielsweise in Form eines Bajonettverschlusses zusammenwirken, wobei beispielsweise eines dieser beiden ersten Schleusenteile 192, 194 oder beide dieser Teile drehbar ausgestaltet sein können, um ein An- und Abkoppeln zu ermöglichen.

Sind das erste Schleusenteil 192 und das erste Sterilschleusenteil 194 miteinander verbunden, so kann sich zwischen der Containertür 170 und der Sterilraumtür 188 ein Zwischenraum ausbilden, welcher in der Regel unsteril ist. Um auch hier eine Mindeststerilität zu gewährleisten, kann beispielsweise die Schleuse 176 und/oder die Sterilschleuse 184 derart ausgestaltet sein, dass dieser Zwischenraum mit minimalem Volumen ausgestattet wird. Alternativ oder zusätzlich können auch Sterilisationseinrichtungen vorgesehen sein, um diesen Zwischenraum beispielsweise vor Öffnen der Containertür 170 und/oder der Sterilraumtür 188 zu sterilisieren. Beispielsweise können zu diesem Zweck wiederum Stutzen für die Einleitung von Sterilisationsmedien, Heizeinrichtungen, Strahlungsquellen oder Ähnliches vorgesehen sein, wobei auf die obige Beschreibung zu Figur 1 verwiesen werden kann. Alternativ oder zusätzlich kann auch ein Vakuumstutzen vorgesehen sein, so dass dieser Zwischenraum kurzfristig evakuiert werden kann, um anschließend beispielsweise mit steriler Luft aus dem Sterilraum 180 befüllt zu werden und/oder mit einem sterilen Inertgas. Auf ähnliche Weise kann beispielsweise auch der Stutzen 140 im Container 110 genutzt werden, um den Innenraum 118 des Containers 110 bzw. den Innenraum 124 des Innencontainers 122 zu evakuieren und/oder mit einem sterilen Inertgas und/oder steriler Atmosphäre zu befüllen.

Alternativ oder zusätzlich zu der genannten Möglichkeit, den Zwischenraum zwischen Containertür 170 und Sterilraumtür 188 beim Ankoppeln des Containers 110 zu sterilisieren, ist in Figur 3 eine Ausführungsform dargestellt, bei welcher der Zwischenraum zwischen der Containertür 170 und der Sterilraumtür 188 praktisch vollständig vermieden wird, indem diese beiden Türen 170, 188 unmittelbar aneinander ankoppeln. Diese unmittelbare Kopplung kann insbesondere auch ermöglichen, dass die Türen 170, 188 nicht getrennt und beispielsweise nacheinander geöffnet werden müssen, sondern gleichzeitig geöffnet werden können. Zu diesem Zweck kann insbesondere die Schleuse 176 neben dem ersten Schleusenteil 192 ein zweites Schleusenteil 196 umfassen, welches auf der Außenseite der Containertür 170 angeordnet ist. Dieses zweite Schleusenteil 196 kann beispielsweise wiederum in Form eines umlaufenden Vorsprungs auf der Außenseite der Containertür 170 ausgestaltet sein, beispielsweise wiederum als ein Teil eines Bajonettverschlusses.

Analog umfasst die Sterilschleuse 184 an der Außenseite der Sterilraumtür 188 ein zweites Sterilschleusenteil 198. Dieses zweite Sterilschleusenteil 198, welches beispielsweise hinter dem Vorsprung des zweiten Schleusenteils 196 eingreifen kann, koppelt somit mit dem zweiten Schleusenteil 196. Beispielsweise kann, wie oben beschreiben, diese Kopplung wiederum einen Bajonettverschluss umfassen.

Dabei kann der Kopplungsmechanismus derart ausgestaltet sein, dass beim Ankoppeln des ersten Schleusenteils 192 an das erste Sterilschleusenteil 194 (beispielsweise durch eine Drehbewegung) gleichzeitig das zweite Schleusenteil 196 an das zweite Sterilschleusenteil 198 angekoppelt wird. Dadurch wird zum einen der Zwischenraum zwischen den beiden Türen 170, 188 vorzugsweise auf ein Minimum reduziert, und es erfolgt eine mechanisch feste, beispielsweise drehfeste Verbindung zwischen den beiden Türen 170, 188. Hierdurch können die Sterilraumtür 188 und die Containertür 170, beispielsweise aus dem Sterilraum 180 heraus, gleichzeitig geöffnet und/oder geschlossen werden. Zu diesem Zweck kann die Containertür 170 beispielsweise einen Bajonettverschluss 200 umfassen, welcher beispielsweise durch ein Drehen der mit der Containertür 170 gekoppelten Sterilraumtür 188 geöffnet bzw. geschlossen werden kann. Dementsprechend sind an die Kopplung zwischen dem zweiten Schleusenteil 196 und dem zweiten Sterilschleusenteil 198 lediglich mechanische Anforderungen zu stellen, nicht hingegen besondere Anforderungen hinsichtlich der Sterilität, da durch diese Kopplung keine mikrobielle Abschirmung, sondern lediglich eine mechanische Kopplung erfolgen muss.

Mittels des in Figur 3 gezeigten Transfersystems 178 kann somit eine Anlieferung bereits sterilisierter pharmazeutischer Behältnisse 112 an einen Abfüllbetrieb erfolgen. Die pharmazeutischen Behältnisse 112 können dann im angekoppelten Zustand vom Sterilraum 180 aus, unmittelbar oder gemeinsam mit den Nestern 128, dem Container 110 entnommen werden. Auch eine vollständige Entnahme eines optionalen Innencontainers 122 ist möglich und bevorzugt. Die pharmazeutischen Behältnisse 112 können dann im Sterilraum 180 befüllt werden. Die nicht mehr benötigten Innencontainer 122 und/oder die nicht mehr benötigten Nester 128 können dann wieder in den Container 110 verbracht werden, die Türen 170, 188 können geschlossen werden, und die Schleuse 176 kann abgekoppelt werden. Anschließend kann der Container 110 wieder beispielsweise zurück zu einem Hersteller der pharmazeutischen Behältnisse 112 verbracht werden, um dort wiederverwendet und beispielsweise wieder mit pharmazeutischen Behältnissen 112 befüllt zu werden. Mittels des dargestellten Transfersystems 178 lässt sich somit der Prozess der Befüllung von Spritzenkörpern erheblich rationalisieren und beschleunigen, da beispielsweise neben einer manuellen Entnahme der pharmazeutischen Behältnisse 112 aus dem Container 110 auch eine halbautomatische oder automatische Entladung möglich ist. Weiterhin lassen sich erhebliche Abfälle einsparen, und der Aufwand für die Entfernung zusätzlicher Versiegelungen (beispielsweise Verschweißungen) lässt sich erheblich reduzieren.

### Bezugszeichenliste

- 110: Container
- 112: pharmazeutische Behältnisse
- 114: Spritzenkörper
- 116: Außenhülle
- 118: Innenraum des Containers
- 120: Vorrichtungen zur Aufnahme des Innencontainers
- 122: Innencontainer
- 124: Innenraum des Innencontainers
- 126: Halterungen für die Aufnahme der Nester
- 128: Nester
- 130: Öffnung zum Beladen des Innencontainers
- 132: Nestkörper
- 134: Öffnungen zur Aufnahme der Spritzenkörper
- 136: Schutzfolie
- 138: Kappe
- 140: Stutzen
- 142: Ventil
- 144: Öffnungen für Sterilisation
- 146: Ventilationsvorrichtung
- 148: Filter
- 150: Öffnung im Container
- 152: Strahlungsquelle
- 154: Temperiervorrichtung
- 156: Steuerung
- 158: Rollen
- 160: Feststell- und Bremsvorrichtung
- 162: Lagerfüße
- 164: Transportösen
- 166: äußere Formprofile
- 168: Vertiefungen
- 170: Containertür
- 172: Containeröffnung
- 174: Verschlussmechanismus
- 176: Schleuse
- 178: Transfersystem
- 180: Sterilraum
- 182: Sterilraumwand
- 184: Sterilschleuse
- 186: Sterilraumöffnung
- 188: Sterilraumtür
- 190: Verriegelungsmechanismus
- 192: erstes Schleusenteil
- 194: erstes Sterilschleusenteil
- 196: zweites Schleusenteil
- 198: zweites Sterilschleusenteil
- 200: Bajonettverschluss der Containertür

## Patentansprüche

1. Wiederverwendbarer Container (110) für den sterilen Transfer pharmazeutischer Behältnisse (112), insbesondere von Spritzenkörpern (114), umfassend eine Außenhülle (116) und einen sterilisierbaren Innenraum (118, 124), wobei der Innenraum (118, 124) mikrobiell gegen die Umgebung des Containers (110) isoliert ist, wobei der Innenraum (118, 124) mindestens eine Halterung (126) für die Aufnahme mindestens eines Nests (128) zur Lagerung einer Mehrzahl von pharmazeutischen Behältnissen (112) aufweist, wobei das Nest (128) herausnehmbar in der Halterung (126) aufnehmbar ist, wobei der Container (110) weiterhin mindestens eine Containertür (170) zum Herausnehmen oder Einführen des Nests (128) in den Innenraum (118, 124) und mindestens eine Schleuse (176) zum Ankoppeln des Containers (110) an einen Sterilraum (180) aufweist, wobei die Schleuse (176) derart ausgestaltet ist, dass nach dem Ankoppeln an den Sterilraum ein Öffnen der Containertür (170) ohne wesentliche Unterbrechung der Sterilität ermöglicht wird.

2. Container (110) nach dem vorhergehenden Anspruch, wobei die Schleuse (176) mindestens ein erstes, mit der Außenhülle (116) außerhalb der Containertür (170) verbundenes Schleusenteil (192) umfasst, wobei das erste Schleusenteil (192) die Containertür (170) zumindest teilweise umgibt.

3. Container (110) nach einem der vorhergehenden Ansprüche, wobei die Schleuse (176) mindestens ein erstes, mit der Außenhülle (116) außerhalb der Containertür (170) verbundenes Schleusenteil (192) und mindestens ein zweites, mit der Containertür (170) verbundenes Schleusenteil (196) umfasst, wobei das erste Schleusenteil (192) an ein erstes, eine Sterilraumtür (188) umgebendes Sterilschleusenteil (194) koppelbar ist und wobei das zweite Schleusenteil (196) an ein zweites, mit der Sterilraumtür (188) verbundenes Sterilschleusenteil (198) koppelbar ist.

4. Container (110) nach einem der vorhergehenden Ansprüche, wobei die Schleuse (176) mindestens einen Bajonettverschluss aufweist.

5. Container (110) nach einem der vorhergehenden Ansprüche, wobei die Containertür (170) mindestens eine auf einer Oberseite und/oder an einer Seitenwand des Containers (110) angeordnete Schwenktür, insbesondere ein Schott, umfasst.

6. Container (110) nach einem der vorhergehenden Ansprüche, weiterhin umfassend mindestens einen in dem Innenraum (118, 124) aufnehmbaren Innencontainer (122), wobei die Halterung (126) zur Aufnahme des Nests (128) zumindest teilweise in dem Innencontainer (122) aufgenommen ist, wobei der Innencontainer (122) und die Containertür (170) derart ausgestaltet sind, dass der Innencontainer (122) reversibel aus dem Innenraum (118, 124) herausnehmbar ist.

7. Container (110) nach dem vorhergehenden Anspruch, wobei der Innencontainer (122) als offener Innencontainer (122) ausgestaltet ist, insbesondere als von fluiden Sterilisationsmedien durchströmbarer Innencontainer (122).

8. Container (110) nach einem der vorhergehenden Ansprüche, wobei die Halterung (126) für die Aufnahme des Nests (128) mindestens eine Schiene zum Einschieben des Nests (128) umfasst.

9. Container (110) nach einem der vorhergehenden Ansprüche, weiterhin umfassend eine Mehrzahl von Nestern (128) zur Lagerung einer Mehrzahl von pharmazeutischen Behältnissen (112).

10. Container (110) nach dem vorhergehenden Anspruch, wobei die Nester (128) mindestens einen im Wesentlichen ebenen Nestkörper (132) umfassen, wobei der im Wesentlichen ebene Nestkörper (132) eine Mehrzahl von Öffnungen (134) zur Aufnahme der pharmazeutischen Behältnisse (112) aufweist.

11. Container (110) nach einem der vorhergehenden Ansprüche, wobei der Container (110) mindestens eine Verbindungsöffnung, insbesondere einen Stutzen (140), zum Einleiten und/oder Ausleiten mindestens eines fluiden Desinfektionsmediums und/oder Sterilisationsmediums in den bzw. aus dem Innenraum (118, 124) umfasst.

12. Container (110) nach einem der vorhergehenden Ansprüche, weiterhin umfassend mindestens eine Ventilationsvorrichtung (146) zur zumindest teilweisen Durchmischung einer Atmosphäre im Innenraum (118, 124).

13. Container (110) nach einem der vorhergehenden Ansprüche, weiterhin umfassend mindestens eine Öffnung zum Einbringen einer Strahlungsquelle (152) in den Innenraum (118, 124).

14. Container (110) nach einem der vorhergehenden Ansprüche, weiterhin umfassend mindestens eine Strahlungsquelle (152) zur Emission radioaktiver Strahlung und/oder elektromagnetischer Strahlung zur Desinfektion und/oder Sterilisation des Innenraums (118, 124).

15. Container (110) nach einem der vorhergehenden Ansprüche, weiterhin umfassend mindestens eine Temperiervorrichtung (154) zum Heizen und/oder Kühlen des Innenraums (118, 124).

16. Container (110) nach einem der vorhergehenden Ansprüche, weiterhin umfassend mindestens einen mikrobiellen Filter (148) zur Filterung von in dem Innenraum (118, 124) umgewälzter Atmosphäre und/oder zur Filterung von in den Innenraum (118, 124) eindringenden fluiden Medien, insbesondere Luft.

17. Container (110) nach einem der vorhergehenden Ansprüche, weiterhin umfassend eine Mehrzahl von Rollen (158), insbesondere Rollen (158) mit Bremseinrichtungen (160), zum Transport des Containers (110).

18. Container (110) nach einem der vorhergehenden Ansprüche, weiterhin umfassend eine Mehrzahl von Lagerfüßen (162) zur stationären Lagerung des Containers (110).

19. Container (110) nach einem der vorhergehenden Ansprüche, wobei der Container (110) äußere Formprofile (166) aufweist, welche derart ausgestaltet sind, dass eine Mehrzahl von Containern (110) stapelbar ist.

20. Container (110) nach einem der vorhergehenden Ansprüche, wobei der Container (110) weiterhin mindestens eine von außen zugängliche Kupplung (164) zum Ankoppeln einer Transporteinrichtung, insbesondere eines Transporthakens, aufweist.

21. Container (110) nach einem der vorhergehenden Ansprüche, wobei der Container (110) und/oder der Innencontainer (122) im Wesentlichen formstabil ausgestaltet sind und mindestens einen der folgenden Werkstoffe aufweisen: ein Metall, insbesondere Edelstahl; eine Keramik; ein Glas; einen Kunststoff, insbesondere einen fluorierten Kunststoff; einen Verbundwerkstoff.

22. Transfersystem (178) für den sterilen Transfer pharmazeutischer Behältnisse (112) in einen Sterilraum (180), umfassend mindestens einen Container (110) nach einem der vorhergehenden Ansprüche, weiterhin umfassend mindestens einen Sterilraum (180), insbesondere einen begehbaren Sterilraum (180), wobei der Sterilraum (180) eine Sterilraumwand (182) mit einer Sterilschleuse (184) umfasst, wobei die Sterilschleuse (184) an die Schleuse (176) des Containers (110) ankoppelbar ist.

23. Transfersystem (178) nach dem vorhergehenden Anspruch, wobei der Sterilraum (180) weiterhin mindestens eine Abfülllinie zum Abfüllen mindestens eines pharmazeutischen Mediums in pharmazeutische Behältnisse (112) aufweist.

24. Verfahren zum Befüllen pharmazeutischer Behältnisse (112) mit mindestens einem pharmazeutischen Medium, insbesondere zum Befüllen von Spritzenkörpern (114), insbesondere unter Verwendung mindestens eines Containers (110) nach einem der vorhergehenden, auf einen Container (110) gerichteten Ansprüche, wobei das Verfahren folgende Schritte umfasst:
- eine Mehrzahl pharmazeutischer Behältnisse (112) wird hergestellt;
- die pharmazeutischen Behältnisse (112) werden sterilisiert;
- die pharmazeutischen Behältnisse (112) werden in mindestens ein Nest (128) zur Lagerung einer Mehrzahl von pharmazeutischen Behältnissen (112) eingebracht;
- das Nest (128) wird in einen wiederverwendbaren Container (110) eingebracht, wobei der Container (110) eine Außenhülle (116) und einen sterilisierbaren Innenraum (118, 124) und mit einer Containertür (170) umfasst, wobei der Innenraum (118, 124) mikrobiell gegen die Umgebung des Containers (110) isoliert ist, wobei der Innenraum (118, 124) mindestens eine Halterung (126) für die Aufnahme des Nests (128) aufweist;
- der Container (110) wird zu einem Sterilraum (180) verbracht und über mindestens eine Schleuse (176) an den Sterilraum (180) angekoppelt, wobei die Schleuse (176) derart ausgestaltet ist, dass nach dem Ankoppeln an den Sterilraum (180) ein Öffnen der Containertür (170) vom Sterilraum (180) her ohne wesentliche Unterbrechung der Sterilität ermöglicht wird;
- das Nest (128) und/oder die pharmazeutischen Behältnisse (112) werden durch die Containertür (170) in den Sterilraum (180) überführt;
- das mindestens eine pharmazeutische Medium wird in die pharmazeutischen Behältnisse (112) eingebracht.

25. Verfahren nach dem vorhergehenden Anspruch, wobei das Einbringen des pharmazeutischen Mediums mittels einer Line-Filler-Anlage oder einer Tray-Filler-Anlage erfolgt.

26. Verfahren nach dem vorhergehenden Anspruch, wobei der Container (110) anschließend von dem Sterilraum (180) entkoppelt wird und zurück zum Ort der Herstellung der pharmazeutischen Behältnisse (112) verbracht wird.
